(19) Europäisches Patentamt
European Patent Office
Office européen des brevets

(11) **EP 2 120 048 B1**

(12) **EUROPEAN PATENT SPECIFICATION**

(45) Date of publication and mention
of the grant of the patent:
**28.01.2015 Bulletin 2015/05**

(51) Int Cl.:
***G01N 33/558*** (2006.01)    ***G01N 33/543*** (2006.01)
***G01N 33/52*** (2006.01)    ***B01L 3/00*** (2006.01)

(21) Application number: **07720917.9**

(22) Date of filing: **23.04.2007**

(86) International application number:
**PCT/CN2007/001344**

(87) International publication number:
**WO 2008/110044 (18.09.2008 Gazette 2008/38)**

(54) **IMMUNE CHROMATOGRAPHIC STRIP DISC FOR MULTIPLE ANALYSIS AND DETECTING METHOD BY USING IT**

IMMUNCHROMATOGRAPHISCHE STREIFENSCHEIBE FÜR DIE MEHRFACHANALYSE SOWIE NACHWEISVERFAHREN UNTER VERWENDUNG DESSELBEN

DISQUE À BANDELETTES IMMUNOCHROMATOGRAPHIQUES POUR UNE ANALYSE MULTIPLE ET PROCÉDÉ DE DÉTECTION UTILISANT CELUI-CI

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **09.03.2007 CN 200710064311**

(43) Date of publication of application:
**18.11.2009 Bulletin 2009/47**

(73) Proprietor: **Institute Of Microbiology And Epidemiology,
Academy Of Military Medical Sciences, Chinese Pla
Fengtai District
Beijing 100071 (CN)**

(72) Inventors:
• **ZHOU, Lei**
**Beijing 100071 (CN)**
• **DU, Zongmin**
**Beijing 100071 (CN)**
• **YANG, Ruifu**
**Beijing 100071 (CN)**
• **HUANG, Huijie**
**Shanghai 201800 (CN)**

(74) Representative: **Stuttard, Garry Philip**
**Urquhart-Dykes & Lord LLP
Tower North Central
Merrion Way
Leeds LS2 8PA (GB)**

(56) References cited:
EP-A2- 1 538 445    WO-A1-2005/031356
WO-A2-2005/116651    CN-A- 1 377 300
CN-A- 1 667 402    DE-U1-202005 011 777
US-A1- 2004 152 206    US-A1- 2005 227 370
US-A1- 2006 008 847    US-B1- 6 203 757
US-B1- 6 573 108    US-B1- 6 740 293

**Description**

**Technical Field**

The present invention belongs to the field of immunologic diagnosis technique, and relates to an immunochromatographic technique for multiple detections. The present invention provides an immunochromatographic test strip plate and an immunochromatographic method for multiple detections by the test strip plate. This makes it possible that test results, whether various analytes exist or not, can be gained by detecting one sample only once.

**Background of the Invention**

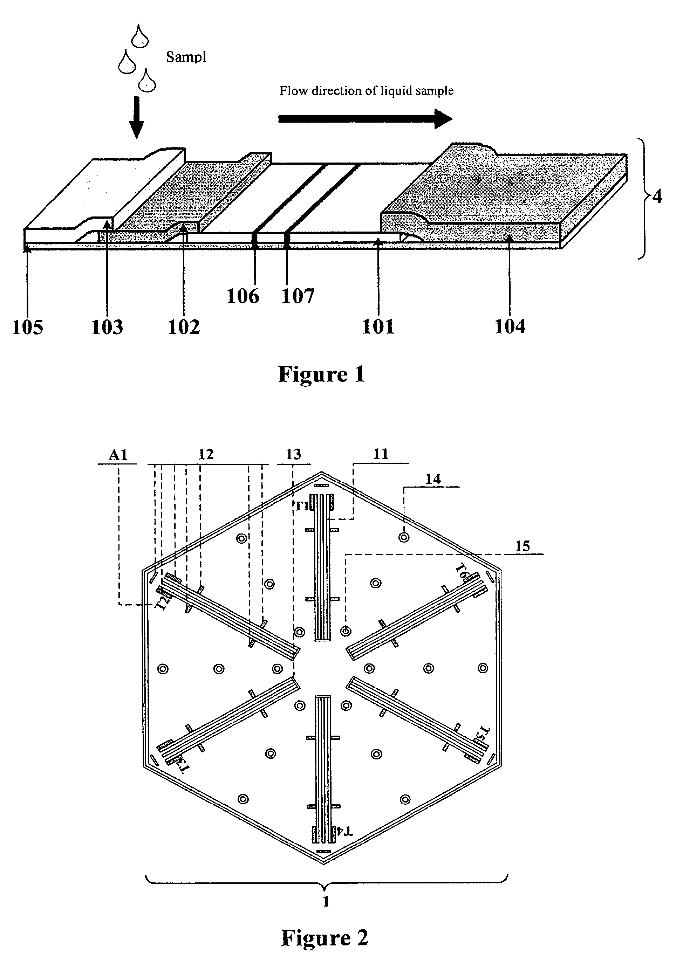

Immunochromatography is a kind of comparatively mature technique for on-site rapid detection. Traditional immunochromatographic test strip 4 is shown in Figure 1. The strip comprises the following components: analytical membrane (mainly nitrocellulose membrane) 101, conjugate pad (mainly glass fiber) 102, sample pad 103 (mainly glass fiber or absorbent paper) and absorbent pad (mainly absorbent paper) 104. The above components are fixed on the sticky substrate 105 with proper overlapping sequence. The overlap of the above components ensures continuity of liquid flow on the strip. When performing the detection, a sample is added to sample pad 103, then enters the conjugate pad 102 through penetration and siphon to redissolve marker-biomolecular conjugates therein. Under the siphon effect of absorbent pad 104, the sample and conjugates leave the conjugate pad 102, enter into the membrane 101 and flow toward the absorbent pad 104 inside the membrane 101. In the processing, specific immunologic reactions will occur between conjugates, target analytes, test line 106 and control line 107 to generate indicative signals. Markers which are commonly used to generate indicative signals include colloid gold, fluorescein, dye, etc. However, any kind of immunochromatographic strip has to follow the detecting mode of one-to-one, namely, only one analyte can be detected in one assay for one sample. This kind of detecting mode is complicated and time-consuming when it's used for screening various target analytes in suspected samples.

US2006/008847 discloses a pinch wall system comprising a sample receiving pad, a test strip and a pinch wall. Each of WO-A-2005/031356 and EP-A-1538445 discloses an immunochromatographic assay system. US6203757B discloses a diagnostic test device with an array of test strips. US2005/227370 discloses a combination body fluid analyte meter and cartridge system. WO-A-2005/116651 discloses a kit for a specific binding assay for the detection of free light chains in untreated urine. DE202005011777U discloses a sample analyte concentration sensor with a housing with a removable fluid impermeable foil strip barrier. None of the aforementioned documents discloses the relationship between the height of the lower fringe and upper fringe of the drainage channel.

One purpose of the invention is to provide an immnochromatographic test strip plate for multiple detections. It is simple and time-saving compared with one-to-one detecting mode in the prior art.

Another purpose of the invention is to provide an immunochromatographic method for multiple detections by the test strip plate of the present invention. This make it possible for a detecting mode of one-to-many, namely, various target analytes can be detected in one assay for one sample simultaneously.

The present invention provides immunochromatographic test strip plate for multiple detections comprising: a base, a upper cover engaged with the base and a drainage piece disposed between the strips on the base and the upper cover, wherein a sample-adding opening is disposed in said upper cover directly opposite the drainage piece, and said sample-adding opening connects with a drainage groove formed by many drainage channels on the underside of the upper cover; wherein several strip stages on the base are opposite to the drainage channels on the upper cover according to their location and number, and the edge of the drainage piece laps to the sample pads adjacent to one end of the sample-adding opening, wherein each drainage channel comprises two upper fringes and a lower fringe and all drainage channels connect sequentially, wherein the inner side of the two upper fringes occlude with the edge of the stage and extend directly to the upside of the base, wherein the relationship between the height of lower fringe $h_2$ and the height of upper fringe $h_1$ of the drainage channel is expressed as follows:

$$h_2 = h_1 - (\text{height of stage} + \text{thickness of sticky substrate of strip} + \text{thickness of sample pad of strip}).$$

According to above said immunochromatographic test strip plate for multiple detections, the shape of said plate for multiple detections may be one of a group including: circle, square, rectangle, diamond, regular polygon. The sample-adding opening is located at the geometric center of one of the above shapes. Several strip stages of the base are arranged in central symmetry or axial symmetry.

According to above said immunochromatographic test strip plate for multiple detections, several sets of fixing stoppers are disposed along the edge of every stage in immunochromatographic plate, and numbering regions are located on each stage near the edge of the plate base.

According to above said immunochromatographic test strip plate for multiple detections, the strip stage consists of three protuberances, and the end adjacent to the symmetric center or axis is sealed by equal-height stoppers.

According to above said immunochromatographic test strip plate for multiple detections, in underside of cover, several sets of pressure pieces are disposed on underside of the upper cover at interval place where the channel of the drainage groove extends outwards. The channel of the drainage groove extends outwards, a result-observing window, an endpoint-indicating window and fixing rivets are also disposed on underside of the upper cover in interval sequence.

According to above said immunochromatographic test strip plate for multiple detections, several rows of female rivets are disposed in the base of the plate and male rivets are correspondingly disposed on underside of the cover.

Specifically, shape of said immunochromatographic test strip plate for multiple detections is one of a group including: circle, diamond and regular polygon, the sample-adding opening is disposed at the geometric center and several strip stages on the base would be arranged in central symmetry.

Moreover, shape of above said strip plate is one of a group including: square and rectangle, the sample-adding opening would be disposed at the symmetric axis and several strip stages on the base would be arranged in axial symmetry.

Acoording to above said immunochromatographic test strip plate for multiple detections, the numbers on the outer side of the lid correspond to those on the base of the disc. In addition, the ID window for marking the serial number of the samples, user holding indication and inserting direction indication are also disposed.

The immunochromatographic method for multiple detections by immunochromatographic test strip plate for multiple detection provided by the invention, which can be used for qualitative detection, comprises the following steps:

Step 1: Assembling the test strip plate by placing different types of the strips on the corresponding stages with different serial numbers.
Step 2: Adding a liquid sample through the sample-adding opening and judge the test endpoint through endpoint-indicating window.
Step 3: Observing and recording the results of the different strips through the result-observing windows.
Step 4: Comparing the test results with standards in order to judge occurrence of certain immunologic reaction and determine existence of certain analyte.

The immunochromatographic method for multiple detection by immunochromatographic test strip plate for multiple detection provided by the invention, which can be used for quantitative detection, comprises the following steps:

Step 1: Assembling the test strip plate by placing different types of the strips on the corresponding stage with different serial numbers.
Step 2: Adding a liquid sample through the sample-adding opening and judge the test endpoint through the endpoint-indicating window.
Step 3: Inserting the plate into detector according to the user holding indication and the inserting direction indication on outside of the upper cover of plate.
Step 4: Powering on the detector to analyze one strip quantitatively through the result-observing window. Test result of the strip is displayed by the apparatus.
Step 5: Rotating or moving the plate to the next numbered strip and powering on the detector again to perform another quantitative analysis.
Step 6: Repeating the step 5 until all strips on the plate are analyzed.
Step 7: Determining existence of certain analyte and its concentration.

The core idea of the invention lies in establishing an immunochromatographic detecting mode for multiple detections by designing a specific immunochromatographic test strip plate, so that various immunochromatographic reactions can occur symmetrically and synchronously in multiple strips for one sample.

Compared with the prior art, the invention has the following technique advantages:

Compared with traditional one-to-one immuno-detecting mode, the invention establishes an immunochromatographic detecting mode for multiple detections by designing a specific immunochromatographic test strip plate so that the existence of various target analytes can be determined in one assay for one sample. Thus, a detection mode of one-to-many is achieved. In the prior art, although the mode of performing multiple detections with several kinds of the strips was mentioned briefly, it does not ultimately resolve the problems that exist necessarily during the combined detection, such as uneven distribution of the liquid sample, non-uniform immunochromatography reaction and liquid sample loss caused by overflow, etc. It makes the combined detection can not be actually achieved. The present invention directly resolves the above problems by means of the special arrangement of the strips, the usage of the drainage piece and the design of the drainage groove on the upper cover of the plate. With these advantages, the synchronous detections which are uniform and prompt can be carried out successfully.

## Description of the Drawings

Figure 1: a graph showing the structure of the strip.
Figure 2: a graph showing the base of the plate.
Figure 3A: a graph showing the underside of the upper cover of the plate.
Figure 3B: a graph showing the structure of the drainage channel in underside of the upper cover of the plate.
Figure 4: a graph showing the outer side of the upper cover of the plate.
Figure 5: a graph showing the assembling process of the plate.
Figure 6: a graph showing the base of the centrally symmetric 10 strips-assembled plate.
Figure 7A: a graph showing the underside of the centrally symmetric 10 strips-assembled plate cover.
Figure 7B: a graph showing the structure of the drainage channel in underside of the centrally symmetric 10 strips-assembled plate cover .
Figure 8: a graph showing the outer side of the centrally symmetric 10 strips-assembled plate cover.
Figure 9: a graph showing the assembling process of the centrally symmetric 10 strips-assembled plate.
Figure 10: a graph showing the base of the axially symmetric 10 strips-assembled plate.
Figure 11A: a graph showing the underside of the upper cover of the axially symmetric 10 strips-assembled disc.
Figure 11B: a graph showing the structure of the drainage channel on the underside of the upper cover of the axially symmetric 10 strips-assembled disc.
Figure 12: a graph showing the outer side of the upper cover of the axially symmetric 10 strips-assembled plate.
Figure 13: a graph showing the assembling process of the axially symmetric 10 strips-assembled plate.

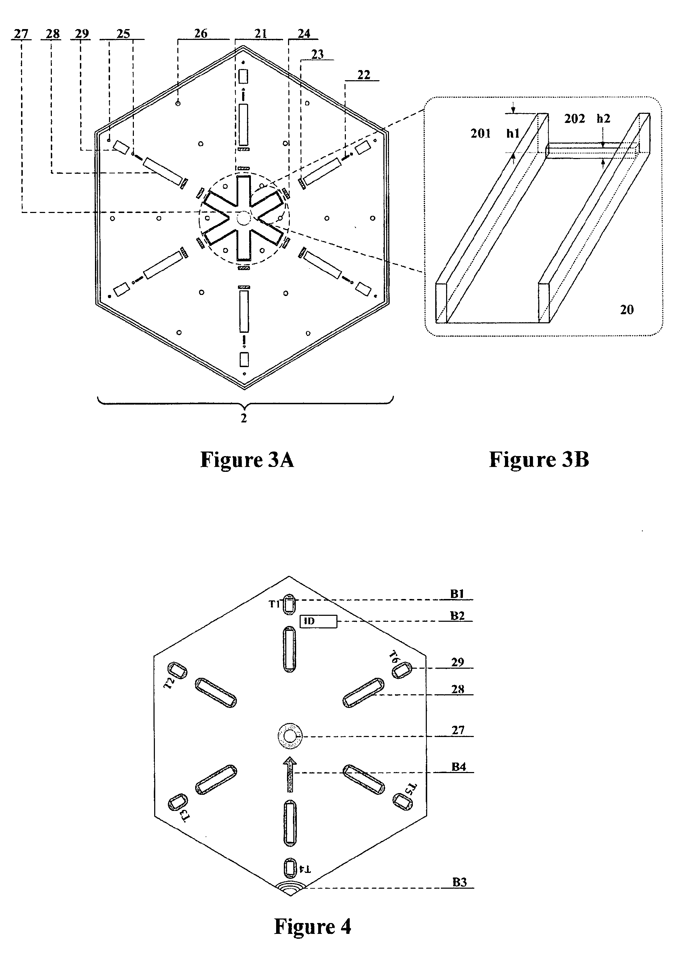

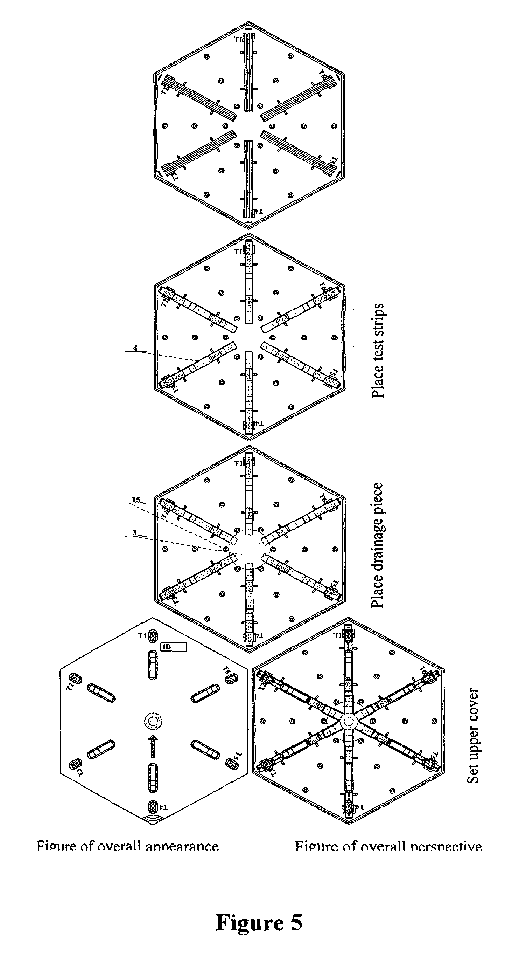

EmbodimentsTest strip plate designed in the present invention comprises four components: a base 1 (see Figure 2, Figure 6, Figure 10), a upper cover 2 (see Figure 3A/3B and Figure 4, Figure 7A/7B and Figure 8, Figure 11A/11B and Figure 12), drainage piece 3 (see Figure 5, Figure 9, Figure 13) and test strips 4 (see Figure 1). Said base 1 and upper cover 2 are fitted to engage for use. Said drainage piece 3 are disposed between the upper cover 2 and the test strips carried on the base 1.

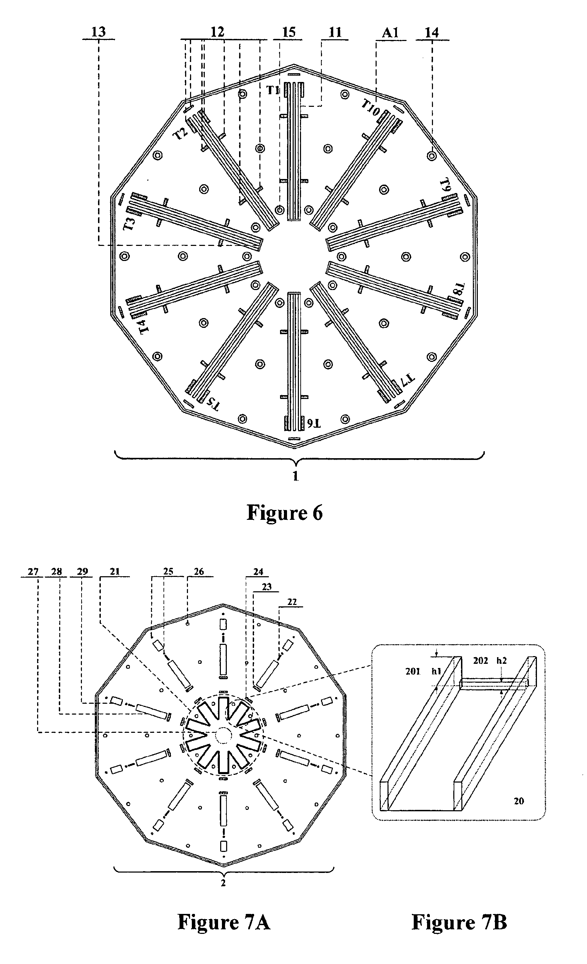

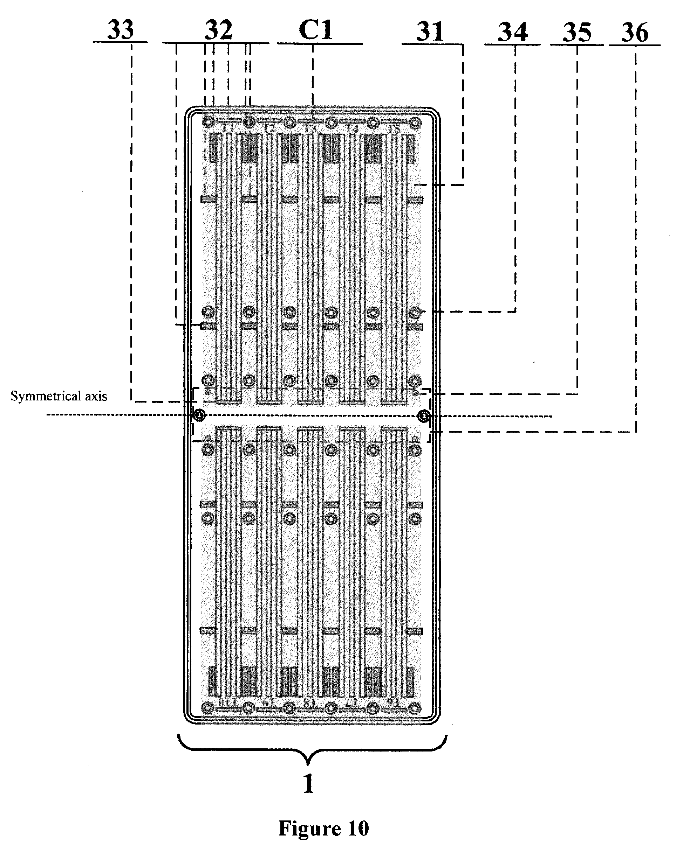

The base 1 contains several strip stages 11 which are arranged in central symmetry (see Figure 2, Figure 6) or axial symmetry (see Figure 10). Several sets of fixing stoppers 12 and equal-height stoppers 13 are disposed along the edge of every stage 11. Numbering region A1 is provided on each stage near the edge of the base 1. Several rows of female rivets 14 are disposed in the base of the disc.

As shown in Figure 2, said strip stage 11 comprises three protuberances, and its end adjacent to the symmetric center or axis is sealed by equal-height stopper 13 to prevent overflow of the liquid sample. Said fixing stopper 12 is configured around the strip stage 11. Said strip stage 11 is combined with the fixing stoppers 12 for carrying and fixing strips, thereby keeping them in central symmetry or axial symmetry as consistent with strip stages 11, with sample pads of test strips gathering at the symmetric center or axis. Said numbering region A1 is provided near the edge of each strip stage 11 for indicating the different types of strips carried on the strip stage 11. Said several rows of female rivets 14 are used to engage with the upper cover 2, wherein the group of female rivets 14 adjacent to the symmetric center or symmetry axis innermostly can also be used as fixed rivets 15 for fixing the drainage piece 3.

See Figure 3 and Figure 4, the upper cover 2 comprises both underside and upside of the upper cover, and consists of the following components: drainage groove 21, several sets of pressure pieces 22, 23, 24, fixing rivets 25, several rows of male rivets 26, sample-adding opening 27, result-observing window 28, endpoint-indicating window 29, numerical code B1, ID window B2, holding indication B3 and inserting direction indication B4. Drainage groove 21, pressure pieces 22,23,24, fixing rivets 25 , male rivets 26 are disposed on underside of the upper cover. Sample-adding opening 26, result-observing window 27 and endpoint-indicating window 28 penetrate through the upper cover 2 from underside to upside. Numerical code B1, ID window B2, holding indication B3 and inserting direction indication B4 are only disposed on upside of the upper cover.

Said drainage groove 21 is formed by integrating the drainage channels 20 corresponding to each strip. As shown in Figure 3B, said drainage channel 20 comprises two upper fringes 201 and one lower fringe 202. The inner sides of said upper fringe 201 occlude with the edge of the stage 11 and extend directly to the upside of the base 1. The relationship between height $h2$ of said lower fringe 202 and height of the upper fringe $h1$ is expressed as follows: $h2=h1-$(height of stage + thickness of sticky substrate of strip + thickness of sample pad of strip). The lower fringe 202 chucks the sample pad 103 tightly. Said upper fringes 201 and lower fringes 202, together with the stage 11, equal-height stoppers 13, the strips 4, the drainage piece 3, upside of the base 1 and underside of the lid of disc 2, define a closed sample pool and the drainage channels corresponding to each strip.

Said three pressure pieces 22,23,24 correspond to the overlapping areas of absorbent pad 104 and analytic membrane 101, conjugate pad 102 and analytic membrane 101, as well as sample pad 103 and the conjugate pad 102 (see Figure 1) in turn, which ensures the continuity of the liquid flow in the strip. The said fixing rivet 25 can penetrate into the absorbent pad 104 of the strip for further stabilizing the strip to prevent it from moving. Said male rivets 26 can interlock with the female rivets 14 on the base 1, which further engage and fix upper cover 2 and base 1. Said sample-adding opening 27 is disposed at the center of cover 2 and is opposite to the drainage piece 3 after the base and cover are engaged together. In the process of detection, the liquid sample is added to the drainage piece 3 through the sample-adding opening 27. Said result-observing window 28 and endpoint-indicating window 29 are arranged in line and are disposed corresponding to the stage 11 of the base 1, wherein the result-observing window 28 is disposed in the middle of the line and opposite to the analytic membrane 101 of the test strip after the base and cover are engaged together. After the assay is completed, the result can be evaluated based on the analytic membrane 101 through the result-observing window 28. Terminal-indicating window 29 is disposed on the portion of the line adjacent to the edge of the upper cover 2 and opposite to the absorbent pad 104 of the strip after the base and upper cover are engaged together. The immunochromatography process can be monitored through endpoint-indicating window during the detection.

Said numerical code B1 on the upper cover, corresponding to the numerical code A1 on the base 1 one to one, is used for indicating different types of the strip. Said ID window B2 can be used for marking the serial number of the detected sample. Said holding indication B3 and inserting direction indication B4 can indicate the direction of inserting the plate into the detector for quantitative detection.

As shown in the Figure 5, the drainage piece 3 is a kind of membrane with large bed volume and uniform microscopic structure. The material of membrane can be glassfiber, filter paper, non-woven fabrics, or etc. The drainage piece 3 is opposite to the drainage groove 21 during assembling, so it can contact the sample pads 103 of each strip and partly overlap the sample pads 103.

The above components are assembled to form the plate of the present invention. The assembling process shown in Figure 5 comprises three steps: placing the test strip, placing the drainage piece and installing the upper cover. Firstly, the specific strips are placed on the specific stage 11 according to the numerical code A1 on the base. The arrangement mode of the stages 11 and the orientating function of the fixing stoppers 12 make the strips gathering in central symmetry or axial symmetry with the sample pads. Then, drainage piece 3 is placed with female rivets 14 on the innermost portion of the base 1 as the fixing rivet 15. The drainage piece 3 overlaps with the sample pads of all strips partly. The large bed volume of the draining piece 3 can prevent the overflow of the liquid sample. Furthermore, the uniform microscopic structure of the drainage piece ensures the uniformity of the sample distribution to each strip. At last, the female rivets 14 in the base 1 interlock with the male rivets 26 on the upper cover 2 to integrate the strip plate in union. In the assembled plate, the sample-adding opening 27, the result-observing windows 28 and the endpoint-indicating windows 29 of the upper cover opposite the drainage piece 3 fixed on the base 1, analytic membranes 101 and absorbent pads 104 of each strip, respectively. Furthermore, the three pressure pieces 22,23,24 opposite the overlapping area of the absorbent pad 104 of each strip and the analytic membrane 101, the overlapping area of the conjugate pad 102 and the analytic membrane 101, as well as the overlapping area of the sample pad 103 and the conjugate pad 102, which ensures the continuity of each chromatographic channels. In order to prevent the overflow of the liquid sample and ensure the uniformity of the chromatographic reaction in each strip, the unique drainage groove 21 is designed on underside of the upper cover 2, including the drainage channels 20 corresponding to each strip. The inner sides of the upper fringes 201 on both sides of the drainage channel 20 occlude with the edge of the stage 11 and extend directly to upside of the base 1. The lower fringe 202 on the middle of the drainage channel 20 chunks the sample pad tightly. In addition, the face-centered end of the stage 11 is sealed by the equal-height stopper 13. Following the buckling and closing of the base and upper cover of plate, the stage 11, equal-height stoppers 13, the strips 4, the drainage piece 3, the drainage groove 21 and upside of the base 1 and underside of the upper cover 2 define a closed sample pool and drainage channels corresponding to each strip, thereby effectively preventing sample loss and ensuring the synchronism and uniformity of absorption of the sample by each strip simultaneously.

In summary, the plate of the invention has the following three technical features:

1. The arrangement of strips in central symmetry or axial symmetry ensures the uniformity of absorption of the sample by each strip;
2. The drainage piece partly overlapping with the sample pad of each strip has a large bed volume to prevent the overflow of the liquid sample. Furthermore, the uniform microscopic structure of the drainage piece ensures the uniformity of the sample distribution;
3. The drainage groove on underside of the upper cover and the base define a closed space for reaction and drainage, and it ensures the uniformity of the reaction in each strip and prevents the overflow of the liquid sample simultaneously.

Shape of above said test strip plate may be one of the group including: circle, square, rectangle, diamond, regular polygon and any other geometrical shapes. Said plate can carry N strips, wherein N is natural number. Therefore, the plate of the invention may have various modifications. The present invention will be described by way of the specific examples, but is not intended to be limited to the following examples.

The detecting method of the invention can be used for the qualitative detection, comprising the following steps:

1. Assembling the test strip plate by placing different types of the strips on the stage with different numbers.
2. Adding the liquid sample through the sample-adding opening and judging the test endpoint through the endpoint-indicating window.
3. Observing and recording the results of the different strips through the result-observing window.
4. Comparing the test results with the standard in order to judge occurrence of certain immunologic reaction and determine existence of certain analytes.

The detecting method of the invention can also be used for the quantitative detection, comprising the following steps:

1: Assembling the test strip plate by placing different types of the strips on the stage with different numbers.
2: Adding the liquid sample through the sample-adding opening and judging the test endpoint through endpoint-indicating window.
3: Inserting the plate into detector according to the user holding indication and the inserting direction indication on outside of the upper cover of plate.
4: Powering on the detector to analyze one strip quantitatively through result-observing window. Test result of the strip is displayed by the apparatus.
5: Rotating or movieng the plate to the next numbered strip and powering on the detector again to perform another quantitative analysis.
6: Repeating the step 5 until all strips of the plate are analyzed.
7: Determine existence of certain analyte and its concentration.

Example 1: The centrally symmetric 6 strips-assembled plate.

Figures 2-5 show the structure and the assembling process of the central symmetric 6 strips-assembled plate. The plate according to this example , which is designed to carry six strips, has a shape of hexagon with its geometric center as symmetric center. All sample pads 103 of the strips point to the symmetric center. The details for the plate according to this example have been described as discussed above with reference to Figures, and its description will be omitted.

Example 2: The centrally symmetric 10 strips-assembled plate.

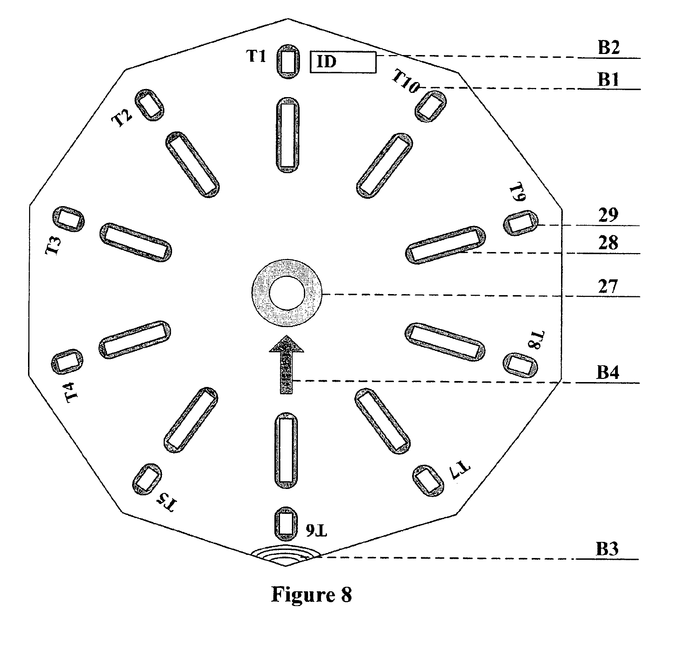

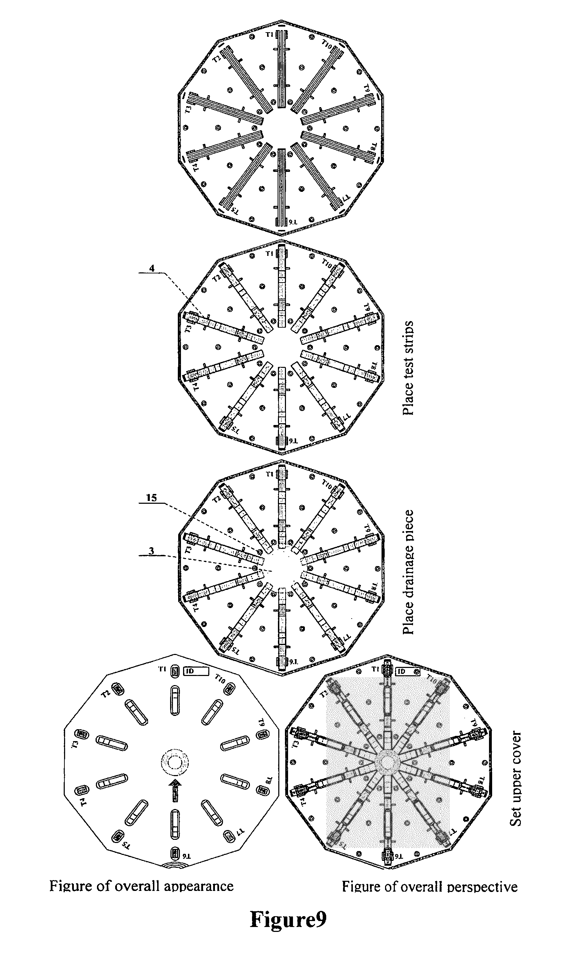

Figures 6-9 are referenced based on above description. The centrally symmetric 10 strips-assembled plate according to this example consists of base 1 (Figure 6), upper cover 2 (Figures 7 and 8), drainage piece 3 and strips 4 (Figure 9). The base 1 includes 10 stages 11, 10 sets of fixing stoppers 12, 10 equal-height stoppers 13, 10 sets of pressure pieces 22,23,24, and 10 rows of female rivets 14, 10 numerical codes A1. The upper cover 2 include drainage groove 21 consisting of 10 drainage channels 20, 10 sets of the pressure pieces 22,23,24, 10 sets of fixing rivets 25, 10 rows of male rivets 26, sample-adding opening 27, 10 result-observing windows 28, 10 teminal-indicating windows 29, 10 numerical codes B1, ID window B2, holding indication B3 and inserting direction indication B4.

The assembling process (Figure 9) comprises the following steps: placing the strips 4, placing the draining piece 3 and installing the upper cover 2. The assembled plate can achieve the uniform reaction of 10 kinds of immuno-ochromatography strips through some special designs. The stages 11 are arranged in central symmetry, and fit with the fixing stoppers 12 and the fixing rivets 25 on the upper cover to make 10 strips arranging in central symmetry gathering toward the center with the sample pads 103. Therefore, with such spatial structure, the uniform immunochromatographic reaction on the various strips 4 can be achieved. The female rivets 14 in innermost portion of the base 1 fit with the male rivets 26 on the upper cover to engage the plate, and they can also be used as fixing rivets 15 to fix the draining piece 3 and make it partly overlap with the sample pad 103 of each strip 4. Consequently, with large bed volume and uniform internal structure, the drainage piece 3 prevent the overflow of the liquid sample and ensure the even distribution of the sample to each strip, resulting in the uniform reaction in different strips. The base 1 and the upper cover 2 of the plate engage together to make the pressure pieces 22,23,24 on underside of the upper cover 2 press on the overlapping areas of the absorbent pad 104 and the analytic membrane 101, the conjugate pad 102 and the analytic membrane 101, as well as the sample pad 103 and the conjugate pad 102. It ensures the continuity of immunochromatographic reaction in the strips. The drainage groove 21 on underside of the upper cover 2 is formed by integrated ten draining channels 20 corresponding to each strip 4. Each drainage channel 20 includes two upper fringes 201 and one lower fringe 202. The inner sides of two upper fringes 201 occlude with the edge of the stage 11 and extend directly to the upside of the base 1. The relationship between height of lower fringe h2 and height of upper fringe h1 is as follows:

$$h2 = h1 - (\text{ height of stage } + \text{ thickness of sticky substrate of strip } + \text{ thickness of sample pad of strip}).$$

The relationship ensures that the upper fringes 201 occlude with the edge of the stage 11 and chuck to the underside of the base 1, and at the same time, the lower fringe 202 chucks to the sample pad 103 tightly. In addition, the face-centered end of stage 11 is sealed by the equal-height stopper 13. Therefore, following the engagement of the base and upper cover of plate, the stage 11, equal-height stoppers 13, the strips 4, the drainage piece 3, the draining groove 21 and the upside of the base 1 and underside of the upper cover 2 define a closed sample pool and immunochromatographic channel, which can ensure that the sample can be distributed to each strip uniformly while preventing loss of the sample due to overflow. During the detection by the assembled plate, the liquid sample is dropped to the drainage piece 3 through the sample-adding opening 27 and then the immune-reaction begins. The process of the immunochromatographic reaction can be monitored through terminal-indicating window 29 opposite the absorbent pad 104 during the detection and the final result can be read through result-observing window 28 opposite the analytic membrane. For the strips that can be analyzed quantitatively by the detector, the holding indication B3 and inserting direction indication B4 on the plate can indicate the direction of the plate inserted in detector.

At last, a synchronous and uniform immunochromatographic reaction can occur on 10 kinds of strips by using the centrally symmetric 10 strips-assembled plate and the final results for interpretation can be shown visually.

Example 3: The axially symmetric 10 strips-assembled disc.

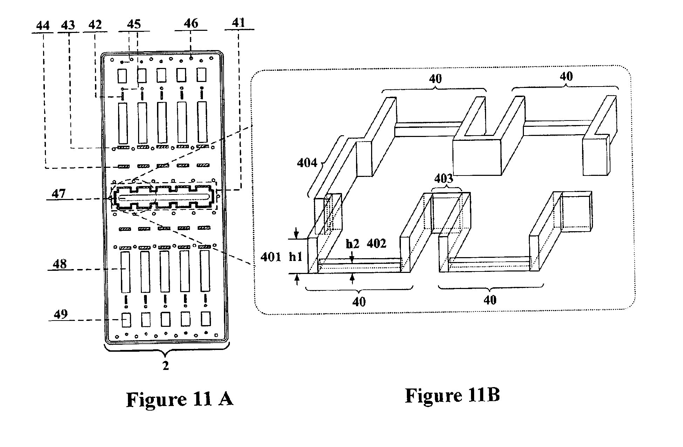

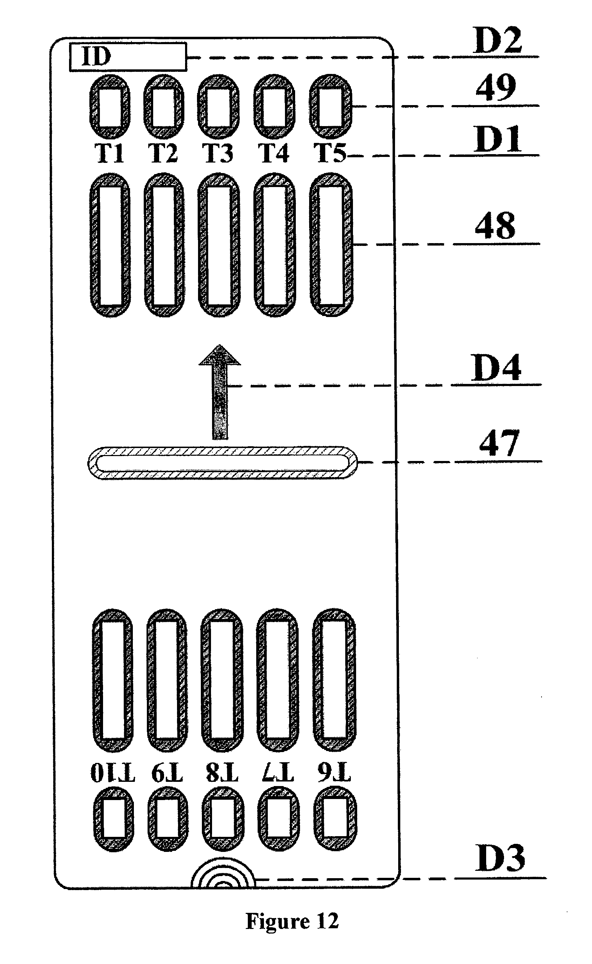

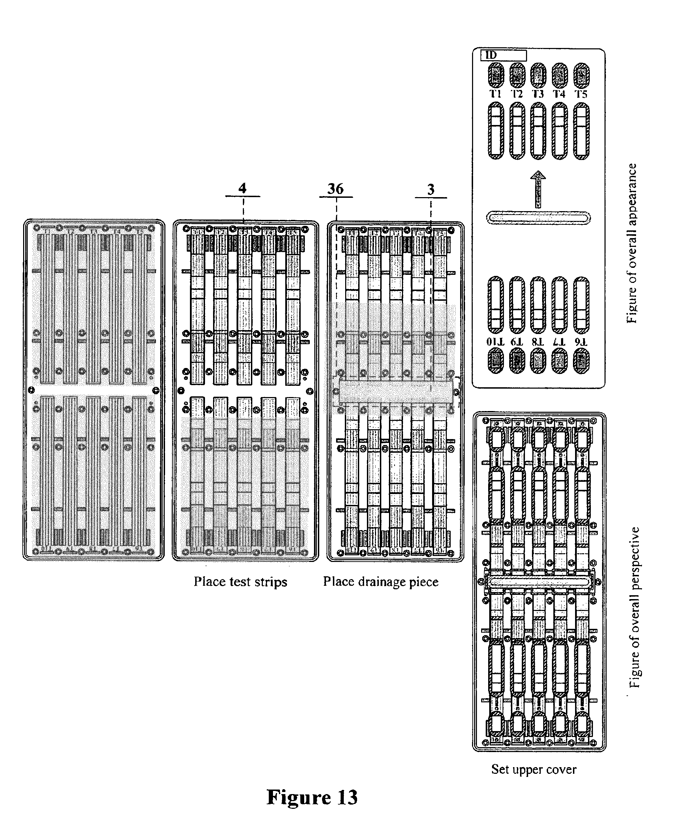

Figures 10-13 are referenced based on above description.. The axially symmetric 10 strips-assembled plate according to this example has a shape of rectangle, and 10 strips are arranged along the axis symmetrically with 5 strips on each side, which makes sample pads 13 of the strips adjacent to the axis. Specifically, the axially symmetric 10 strips-assembled disc consists of base 1 (Figure 10), upper cover 2 (Figures 11 and 12), drainage piece 3 and strips 4 (Figure 13). The base 1 includes stage 31, fixing stoppers 32, equal-height stoppers 33, female rivets 34, male rivets 35, and numerical codes C1. The upper cover 2 include drainage groove 41, pressure pieces 42,43,44, fixing rivets 45, male rivets 46, sample-adding opening 47, result-observing window 48, endpoint-indicating window 49, numerical codes D1, ID window D2, holding indication D3, inserting direction indication D4.

The assembling procedure (Figure 13) includes the following steps: placing the strip 4, placing the drainage piece 3, installing the upper cover 2. The assembled plate can achieve the uniform immunochromatographic reaction of 10 kinds of strips through some special designs. The stages 31 are arranged in axial symmetry, and fit with the fixing stoppers 32 and the fixing rivets 45 on the upper cover to make 10 strips arranging in axial symmetry gathering toward the symmetric axis with the sample pad. Therefore, with such spatial structure, the uniform immunochromatographic reaction on the various strips can be achieved. The female rivets 34 in the base fit with the male rivets 46 on the upper cover to engage the plate, and two female rivets 34 disposed on the symmetric axis and four male rivets 35 can also be used as fixing rivets 36 to fix the drainage piece 3 and make it partly overlap the sample pad 103 of each strip. Consequently, with large bed volume and uniform internal structure, the drainage piece 3 prevent the overflow of the liquid sample and ensure the even distribution of the sample to each strip, resulting in the uniform reaction on the different strips. The base 1 and the upper cover 2 of plate engage together to make the pressure pieces 42,43,44 on underside of upper cover press on the overlapping areas of the absorbent pad 104 and the analytic membrane 101, the conjugate pad 102 and the analytic membrane 101, as well as the sample pad 103 and the conjugate pad 102, which ensures the continuity of immunochromatographic reaction in the strips. The draining groove 41 on underside of the upper cover consists of integrated ten draining channels 40 corresponding to each strip. Each drainage channel 40 includes two upper fringes 401 and one lower fringe 402. Every two drainage channels 40 disposed on the same side of axis are linked by the connection board 403, while two drainage channels 40 disposed on the different side of axis are linked by the connection board 404. The inner sides of two upper fringes occlude with the edge of the stage 31 and extend directly to the underside of the base. The relationship between height of lower fringe h2 and height of upper fringe h1 is as follows:

$$h2=h1-(\text{ height of stage } + \text{ thickness of sticky substrate of strip } + \text{ thickness of sample pad of strip}).$$

The relationship ensures that upper fringes 401 occlude with the stage 31 and chuck to the underside of the base 1, and at the same time, the lower fringe 402 chucks to the sample pad 103 tightly. In addition, the face-axis end of stage 31 is sealed by the equal-height stopper 33. Therefore, following the engagement of the base and upper cover of plate, the stage 31, equal-height stoppers 33, the strips 4, the drainage piece 3, the drainage groove 41 and the upside of the base 1 and underside of the upper cover 2 define a whole closed sample pool and immunochromatographic channel, which can ensure that the sample can be distributed to each strip uniformly while preventing loss of the sample due to overflow. During the detection by the assembled plate, the liquid sample is dropped to the drainage piece 3 through the sample-sdding opening 47 and then the immune-reaction begins.

The process of the immunochromatographic reaction can be monitored through endpoint-indicating window 49 opposite the absorbent pad 103 during the detection and the final result can be read through result-observing window 48 opposite the analytic membrane. For the strips that can be analyzed quantitatively by the detector, the holding indication D3 and the inserting direction indication D4 on the plate can indicate the direction of the plate inserted in detector.

At last, a synchronous and uniform immunochromatographic reaction can occur on 10 kinds of strips by using the axially symmetric 10 strips-assembled plate. The final results for interpretation can be shown visually.

**Claims**

1. An immunochromatographic test strip plate for multiple detections comprising: a base, a upper cover engaged with the base and a drainage piece disposed between the strips on the base and the upper cover, wherein a sample-adding opening is disposed in said upper cover directly opposite the drainage piece, and said sample-adding opening connects with a drainage groove formed by many drainage channels on the underside of the upper cover; wherein several strip stages on the base are opposite to the drainage channels on the upper cover according to their location and number, and the edge of the drainage piece laps to the sample pads adjacent to one end of the sample-adding opening, wherein each drainage channel comprises two upper fringes and a lower fringe and all drainage channels connect sequentially, wherein the inner side of the two upper fringes occlude with the edge of the stage and extend directly to the upside of the base, wherein the relationship between the height of lower fringe h2 and the height of upper fringe h1 of the drainage channel is expressed as follows:

$$h2=h1-(\text{height of stage } + \text{ thickness of sticky substrate of strip } + \text{ thickness of sample pad of strip}).$$

2. The immunochromatographic test strip plate for multiple detections according to claim 1, wherein the shape of said test strip plate is any geometric shape of a group including: circle, square, rectangle, diamond and regular polygon, wherein the sample-adding opening is disposed at the geometric center of one of the said shapes and the strip stages on the base are arranged in central symmetry or axial symmetry.

3. The immunochromatographic test strip plate for multiple detections according to claim 1 or 2, wherein several sets of fixing stoppers are disposed along the edge of every strip stage, and numbering regions are provided on each stage at the place near the edge of the base.

4. The immunochromatographic test strip plate for multiple detections according to any of claims 1 to 3, wherein the strip stage comprises three protuberances, and its end adjacent to the symmetric center or axis is sealed by equal-height stoppers.

5. The immunochromatographic test strip plate for multiple detections according to any of claims 1 to 4, wherein several sets of pressure pieces are disposed on the underside of the upper cover at interval place where the channel of the drainage groove extends outwards, and a result-observing window, an endpoint-indicating window and fixing rivets are disposed on underside of the upper cover in interval sequence.

6. The immunochromatographic test strip plate for multiple detections according to any of claims 1 to 5, wherein several rows of female rivets in the base correspond to male rivets on the underside of the upper cover.

7. The immunochromatographic test strip plate for multiple detections according to any of claims 1 to 6, wherein numbers on the upside of the upper cover correspond to those on the base and an ID window used for marking the serial number of detected samples, a user holding indication and a inserting direction indication are disposed on upside of the upper cover.

8. An immunochromatographic method for multiple detections by an immunochromatographic test strip plate according to any of claims 1 and 5-7 is **characterised in** a qualitative detection comprising the following steps:

    1) Assembling the test strip plate by placing different types of strips on the corresponding stage with different numbers,
    2) Adding a liquid sample through the sample-adding opening and judging test endpoint through endpoint-indicating window,
    3) Observing and recording results of the different strips through result-observing windows, and
    4) Comparing the test results with standard in order to judge occurrence of certain immunologic reactions and determine existence of certain analytes.

9. An immunochromatographic method for multiple detections according to claim 8 is **characterised in** a quantitative detection comprising the following steps:

    1) Assembling the test strip plate by placing different types of strips on the corresponding stage with different numbers,
    2) Adding a liquid sample through the sample-adding opening and judging test endpoint through the endpoint indicating window,
    3) Inserting the plate into detector according to the user holding indication and the inserting direction indication on outside of the upper cover of plate,
    4) Powering on the detector to analyze one strip quantitatively through result-observing window, and test result of the strip is displayed by the apparatus.,
    5) Rotating or movieng the plate to the next strip and powering on the detector again to perform another quantitative analysis,
    6) Repeating step 5) until all strips on the plate are analyzed, and
    7) Determining existence of certain analyte and its concentration.

## Patentansprüche

1. Immunchromatographische Teststreifenplatte für mehrfache Detektionen, umfassend: eine Basis, eine obere Abdeckung, die mit der Basis eingerastet ist, und ein Drainagestück, welches zwischen den Streifen auf der Basis und der oberen Abdeckung angeordnet ist, worin eine Probenzugabeöffnung in der oberen Abdeckung direkt gegenüber des Drainagestücks angeordnet ist, und sich die Probenzugabeöffnung mit einer Drainagerinne verbindet, welche durch viele Drainagekanäle auf der Unterseite der oberen Abdeckung gebildet wird; worin verschiedene Streifenebenen auf der Basis gemäß ihrer Verortung und Anzahl gegenüberliegend zu den Drainagekanälen auf der oberen Abdeckung sind, und die Kante des Drainagestücks zu den Probeplättchen, angrenzend an ein Ende der Probenzugabeöffnung, überlappt, worin jeder Drainagekanal zwei obere Ränder und einen unteren Rand umfasst, und alle Drainagekanäle sich sequentiell verbinden, worin die innere Seite der zwei oberen Ränder die Kante der Ebene verdecken und sich direkt auf die Oberseite der Basis erstrecken, wobei die Beziehung zwischen der Höhe des unteren Rands h2 und der Höhe des oberen Rands h1 des Drainagekanals wie folgt ausgedrückt ist:

$$h2 = h1 - (\text{Höhe der Ebene + Dicke des klebrigen Substrats des Streifens + Dicke des Probeplättchens des Streifens}).$$

2. Immunchromatographische Teststreifenplatte für mehrfache Detektionen gemäß Anspruch 1, wobei die Form der Teststreifenplatte eine beliebige geometrische Form aus einer Gruppe hat, welche beinhaltet: Kreis, Quadrat, Rechteck, Diamant und regelmäßiges Polygon, worin die Probenzugabeöffnung am geometrischen Zentrum von einer der Formen angeordnet ist, und die Streifenebenen auf der Basis in zentraler Symmetrie oder axialer Symmetrie ausgerichtet sind.

3. Immunchromatographische Teststreifenplatte für mehrfache Detektionen gemäß Anspruch 1 oder 2, worin mehrere Sätze von Fixierstopfen entlang der Kante von jeder Streifenebene angeordnet sind, und Nummerierungsregionen auf jeder Ebene an der Stelle nahe der Kante der Basis bereitgestellt sind.

4. Immunchromatographische Teststreifenplatte für mehrfache Detektionen gemäß einem der Ansprüche 1 bis 3, worin die Streifenebene drei Vorsprünge umfasst, und ihr Ende, welches an das Symmetriezentrum oder die Symmetrieachse angrenzt, durch Stopfen gleicher Höhe versiegelt ist.

5. Immunchromatographische Teststreifenplatte für mehrfache Detektionen gemäß einem der Ansprüche 1 bis 4, worin verschiedene Sätze von Druckstücken auf der Unterseite der oberen Abdeckung an einer Intervallstelle angeordnet sind, wo der Kanal der Drainagerinne sich nach außen erstreckt, und ein Ergebnisbeobachtungsfenster, ein Endpunkt-Anzeigefenster, und Fixiernieten auf der Unterseite der oberen Abdeckung in Intervallfolge angeordnet sind.

6. Immunchromatographische Teststreifenplatte für mehrfache Detektionen gemäß einem der Ansprüche 1 bis 5, worin verschiedene Reihen von weiblichen Nieten in der Basis männlichen Nieten auf der Unterseite der oberen Abdeckung entsprechen.

7. Immunchromatographische Teststreifenplatte für mehrfache Detektionen gemäß einem der Ansprüche 1 bis 6, worin Zahlen auf der Oberseite der oberen Abdeckung denjenigen auf der Basis entsprechen, und ein ID-Fenster, verwendet zur Markierung der Seriennummer detektierter Proben, eine Benutzer-Haltungsanzeige, und eine Einsetzungsrichtungsanzeige auf der Oberseite der oberen Abdeckung angeordnet sind.

8. Immunchromatographisches Verfahren für mehrfache Detektionen durch eine immunchromatographische Teststreifenplatte gemäß einem der Ansprüche 1 und 5-7, **gekennzeichnet durch** eine qualitative Detektion, welche die folgenden Schritte umfasst:

   1) Zusammensetzen der Teststreifenplatte **durch** Platzieren verschiedener Arten von Streifen auf die entsprechende Ebene mit unterschiedlichen Nummern,
   2) Hinzufügen einer flüssigen Probe **durch** die Probenzugabeöffnung und Beurteilen des Testendpunkts **durch** das Endpunkt-Anzeigefenster,
   3) Beobachten und Aufnehmen der Ergebnisse der verschiedenen Streifen **durch** Ergebnisbeobachtungsfenster, und
   4) Vergleichen der Testergebnisse mit Standard, um das Auftreten von bestimmten immunologischen Reaktionen zu beurteilen, und das Vorliegen bestimmter Analyte zu bestimmen.

9. Immunchromatographisches Verfahren für mehrfache Detektionen gemäß Anspruch 8, **gekennzeichnet durch** eine quantitative Detektion, welche die folgenden Schritte umfasst:

   1) Zusammensetzen der Teststreifenplatte **durch** Platzieren verschiedener Arten von Streifen auf die entsprechende Ebene mit unterschiedlichen Nummern,
   2) Hinzufügen einer flüssigen Probe **durch** die Probenzugabeöffnung und Beurteilen des Testendpunkts **durch** das Endpunkt-Anzeigefenster,
   3) Einsetzen der Platte in einen Detektor gemäß der Benutzer-Haltungsanzeige und der Einsetzungsrichtungsanzeige auf der Außenseite der oberen Abdeckung der Platte,
   4) Einschalten des Detektors, um einen Streifen quantitativ **durch** das Ergebnisbeobachtungsfenster zu analysieren, und Anzeigen des Testergebnisses des Streifens durch die Vorrichtung,
   5) Drehen oder Bewegen der Platte zum nächsten Streifen, und erneutes Einschalten des Detektors, um eine weitere quantitative Analyse durchzuführen,
   6) Wiederholen von Schritt 5) bis alle Streifen auf der Platte analysiert sind, und
   7) Bestimmen des Vorliegens eines bestimmten Analyts und seiner Konzentration.

**Revendications**

1. Plaquette de bandelettes réactives immunochromatographiques pour détections multiples comprenant : une base, un couvercle supérieur mis en prise avec la base et une pièce de drainage disposée entre les bandelettes sur la base et le couvercle supérieur, dans laquelle une ouverture d'ajout d'échantillon est disposée dans ledit couvercle supérieur directement opposé à la pièce de drainage, et ladite ouverture d'ajout d'échantillon se raccorde avec une rainure de drainage formée par de nombreux canaux de drainage sur la face inférieure du couvercle supérieur ; dans laquelle plusieurs étages de bandelettes sur la base sont opposés aux canaux de drainage sur le couvercle supérieur selon leur emplacement et leur nombre, et le bord de la pièce de drainage dépasse des tampons d'échantillon adjacents à une extrémité de l'ouverture d'ajout d'échantillon, dans laquelle chaque canal de drainage comprend deux franges supérieures et une frange inférieure et tous les canaux de drainage se raccordent en séquence, dans laquelle le côté interne des deux franges supérieures se ferme avec le bord de l'étage et s'étend directement vers la face supérieure de la base, dans laquelle la relation entre la hauteur de la frange inférieure h2 et la hauteur de la frange supérieure h1 du canal de drainage est exprimée de la manière suivante :

```
manière suivante :
        h2  =  h1  (hauteur  de  l'étage  +  épaisseur  du
substrat  collant  de  la  bandelette  +  épaisseur  du  tampon
d'échantillon de la bandelette).
```

2. Plaquette de bandelettes réactives immunochromatographiques pour détections multiples selon la revendication 1, dans laquelle la forme de ladite plaquette de bandelettes réactives a une forme géométrique quelconque d'un groupe comprenant : le cercle, le carré, le rectangle, le diamant et le polygone régulier, dans laquelle l'ouverture d'ajout d'échantillon est disposée au centre géométrique de l'une desdites formes et les étages de bandelette sur la base sont agencés en symétrie centrale ou symétrie axiale.

3. Plaquette de bandelettes réactives immunochromatographiques pour détections multiples selon la revendication 1 ou 2, dans laquelle plusieurs ensembles de butées de fixation sont disposés le long du bord de chaque étage de bandelette, et des régions de numérotation sont prévues sur chaque étage à l'endroit situé à proximité du bord de la base.

4. Plaquette de bandelettes réactives immunochromatographiques pour détections multiples selon l'une quelconque des revendications 1 à 3, dans laquelle l'étage de bandelette comprend trois protubérances, et son extrémité adjacente au centre ou à l'axe de symétrie est scellée par des butées de hauteur égale.

5. Plaquette de bandelettes réactives immunochromatographiques pour détections multiples selon l'une quelconque des revendications 1 à 4, dans laquelle plusieurs ensembles de pièces de pression sont disposés sur la face inférieure du couvercle supérieur à chaque endroit où le canal de la rainure de drainage s'étend vers l'extérieur, et une fenêtre d'observation de résultat, une fenêtre d'indication de fin et des rivets de fixation sont disposés sur la face inférieure du couvercle supérieur dans chaque séquence.

6. Plaquette de bandelettes réactives immunochromatographiques pour détections multiples selon l'une quelconque des revendications 1 à 5, dans laquelle plusieurs rangées de rivets femelles dans la base correspondent aux rivets mâles sur la face inférieure du couvercle supérieur.

7. Plaquette de bandelettes réactives immunochromatographiques pour détections multiples selon l'une quelconque des revendications 1 à 6, dans laquelle les numéros sur la face supérieure du couvercle supérieur correspondent à ceux sur la base et une fenêtre ID utilisée pour marquer le numéro de série des échantillons détectés, une unité de support d'utilisateur et une indication de direction d'insertion sont disposées sur la face supérieure du couvercle supérieur.

8. Procédé immunochromatographique pour détections multiples par une plaquette de bandelettes réactives immunochromatographiques selon l'une quelconque des revendications 1 et 5 à 7, **caractérisé par** une détection qualitative comprenant les étapes suivantes consistant à :

1) assembler la plaquette de bandelettes réactives en plaçant différents types de bandelettes sur l'étage correspondant avec différents numéros,

2) ajouter un échantillon liquide par l'ouverture d'ajout d'échantillon et estimer la fin de l'essai par la fenêtre d'indication de fin,

3) observer et enregistrer les résultats des différentes bandelettes par les fenêtres d'observation de résultat, et

4) comparer les résultats d'essai avec la norme afin d'estimer l'occurrence de certaines réactions immunologiques et déterminer l'existence de certains analytes.

9. Procédé immunochromatographique pour détections multiples selon la revendication 8, **caractérisé par** une détection quantitative comprenant les étapes suivantes consistant à :

1) assembler la plaquette de bandes réactives en plaçant différents types de bandelettes sur l'étage correspondant avec des numéros différents,

2) ajouter un échantillon liquide par l'ouverture d'ajout d'échantillon et estimer la fin de l'essai par la fenêtre d'indication de fin,

3) insérer la plaquette dans le détecteur selon l'indication de support d'utilisateur et l'indication de direction d'insertion sur l'extérieur du couvercle supérieur de la plaquette,

4) mettre en marche le détecteur pour analyser une bandelette du point de vue quantitatif par la fenêtre d'observation de résultat et le résultat d'essai de la bandelette est affiché par l'appareil,

5) faire tourner ou déplacer la plaquette jusqu'à la bandelette suivante et remettre en marche le détecteur pour réaliser une autre analyse quantitative,

6) répéter l'étape 5) jusqu'à ce que toutes les bandelettes sur la plaquette soient analysées, et

7) déterminer l'existence d'un certain analyte et sa concentration.

Sampl

Flow direction of liquid sample

106 107

105    103    102    101    104

4

**Figure 1**

A1    12    13    11

14

15

T1

T2

T6

T3

T5

T4

1

**Figure 2**

**Figure 3A**

**Figure 3B**

**Figure 4**

Place test strips

Place drainage piece

Set upper cover

Figure of overall appearance

Figure of overall perspective

# Figure 5

**Figure 6**

**Figure 7A**                    **Figure 7B**

**Figure 8**

Place test strips

Place drainage piece

Set upper cover

Figure of overall appearance

Figure of overall perspective

# Figure9

Figure 10

**Figure 11 A**

**Figure 11B**

Figure 12

**4**  **36**  **3**

Place test strips    Place drainage piece

Figure of overall appearance

Figure of overall perspective

Set upper cover

# Figure 13

**EP 2 120 048 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2006008847 A
- WO 2005031356 A
- EP 1538445 A
- US 6203757 B
- US 2005227370 A
- WO A A
- WO 2005116651 A
- DE 202005011777 U